(19) **Europäisches Patentamt / European Patent Office / Office européen des brevets**

(11) **EP 3 654 050 A1**

(12) **EUROPEAN PATENT APPLICATION**

(43) Date of publication:
20.05.2020 Bulletin 2020/21

(51) Int Cl.:
*G01R 33/54* (2006.01)   *G01R 33/385* (2006.01)

(21) Application number: 18206297.6

(22) Date of filing: 14.11.2018

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Siemens Healthcare GmbH**
**91052 Erlangen (DE)**

(72) Inventors:
• **Feiweier, Thorsten**
  **91099 Poxdorf (DE)**
• **Grübel, Sören**
  **91052 Erlangen (DE)**
• **Köhler, Michael**
  **90408 Nürnberg (DE)**
• **Lenz, Helmut**
  **90522 Oberasbach (DE)**

(54) **OPTIMIZED USE OF A SYSTEM COMPONENT IN AN IMAGING SYSTEM**

(57)   The invention relates to a method for determining a use of a system component (31-33) in an imaging system (1), the imaging system comprising a primary side (21) configured to provide power to the system component and a secondary side (30) comprising the system component (31-33) which uses the power provided by the primary side during the image sequence. The method comprises determining the use of the system component (31-33) during an imaging sequence, determining a time averaged power provided by the primary side during the imaging sequence, determining a maximum time averaged power that can be provided by the primary side (21) until a temperature limit is reached on the primary side. Furthermore it is determined whether the time averaged power is smaller than the maximum time averaged power.

FIG 2

$$P_{pri} \sim U_{pri}, I_{pri} \quad P_{sek} = P_x + P_y + P_z$$

EP 3 654 050 A1

# EP 3 654 050 A1

**Description**

The invention relates to a method for determining a use of a system component in an imaging system in which at least one image of an object under examination is generated during the imaging sequence. Furthermore, the corresponding imaging system is provided, a computer program comprising program code and a carrier comprising the computer program.

For the generation of images of an object under examination such as a patient, system components of the imaging systems are used. An example of such a system component may be the amplifier used to generate magnetic field gradients applied in an MR system, the RF amplifier used in an MR system, the power generating unit in an imaging system using x-rays etc. All these system components have some physical limits as the power required to drive the system components is limited and as the power may overheat the system component or any other component involved. There is a strong need to provide cost effective imaging systems and as a consequence the system components used in the imaging system get designed to an increasing degree such that they exhibit more severe limitations regarding their thermal endurance. This, however, might go along with either a decreased image quality or, in order to maintain a desired quality, with longer acquisition times.

The control of the system components in the medical imaging systems is usually designed such that the limitations of the system components are considered on the basis of empirical values. In an MR imaging system this means that there is a system-specific reference gradient amplitude that can be applied during the imaging sequence. This limit of the gradient amplitude is determined such that the MR system is capable of using the gradient amplitude over longer time periods. These reference values of the gradient amplitudes are typically not directly related to the different hardware components that are required to generate the gradient fields. Depending on the imaging sequence used, different components might become the limiting factor. Based on the empirical values used for specifying the reference gradient amplitudes only, an exact prediction of the heating of the different hardware components used in the MR imaging system is not possible, which entails a more conservative gradient utilization. This, however, will either sacrifice image quality or require longer acquisition times to generate an MR image of a desired quality. Otherwise, it is possible that the heat generated by the power needed to operate the system component stops a running imaging sequence so that the latter has to be repeated with a relaxed utilization of the system component.

DE 10 2008 015 261 B4 and DE 10 2013 204 310 A1 describe methods in which the load of the different hardware components is determined in detail using a model in order to determine an optimized imaging sequence.

This approach, however, is based on a detailed and time-resolved knowledge of the heating characteristic of the hardware component. Only when this detailed knowledge is available, it is possible to estimate and compare the current induced heat against a thermal limit. In many cases, however, the complexity of individual components and their interactions are such that a model for the time-resolved heating characteristic based on the actual utilization does not exist. Even in cases where those models are known, the necessary consideration of manufacturing tolerances in these models typically leads to a very conservative use of the system components. Furthermore, these models have to know the initial state of the hardware components, which might turn out not being always available.

In summary the above mentioned examples show that at present a use of the system components in an imaging system is not optimized.

Summary

Accordingly a need exists to provide a possibility to provide an optimized use of a system component in an imaging system in view of a power or current induced heating.

This need is met by the features of the independent claims. Further aspects are described in the dependent claims.

According to a first aspect a method for determining a use of a system component in an imaging system is provided in which at least one image of an object under examination is generated during the imaging sequence. The imaging system comprises a primary side or supply side configured to provide power to the system component resulting in a thermal load on the primary side which must not exceed a predefined temperature limit. The imaging system furthermore comprises a secondary side comprising the system component which uses the power provided by the primary side during the imaging sequence. According to one step of the method the utilisation of the system component during the imaging sequence is determined and a time averaged power provided by the primary side during the imaging sequence is determined with the determined utilisation of the system component. Furthermore, a maximum time averaged power is determined that can be supplied by the primary side over a duration of at least one imaging sequence while not exceeding the predefined temperature limit. Furthermore, it is determined whether the time averaged power is smaller than the maximum time averaged power. When the time averaged power is not smaller than the maximum time averaged power, the use of the system component during the imaging sequence is adapted until the time averaged power is smaller than the maximum time averaged power.

2

The utilisation of the system component is determined by determining the time averaged power without using any initial states of the system component. Furthermore, the utilisation of the system component may be determined without the exact knowledge of a relationship of how the use of the system component during the imaging sequence influences a heating of the primary side.

The method can be based on the assumption that a first time constant which describes the power induced heating of the primary side when power is fed to the system component is actually much longer than the time period in which an output parameter or output quantity of the system component gets switched during the imaging sequence. As the time constant describing the power induced heating is typically longer than the time period in which the system component gets switched and needs the power, it is possible to consider an average load or power of the supply side to determine whether the maximum load or power is exceeded or not.

It is possible that an optimized imaging sequence is determined based on the comparison of the time averaged power to the maximum time averaged power such that either the time needed to generate the at least one image or an image quality parameter of the at least one image such as a signal-to-noise ratio, or a contrast-to-noise ratio in a certain region of interest is optimized.

With the determination of the power induced heating based on the average power the imaging sequence can be adapted such that the system component is used in such a way that a heating of the primary side below its maximum value is obtained and the heating may be closer to the maximum value of the temperature limit than obtained with the methods of the prior art, thus improving the utilisation of the system component.

For determining the time averaged power it is possible to determine an average of a parameter representing the heating of the primary side when power is provided to the system component, and when the maximum of the time averaged power is determined it is possible to determine a maximum of the average of the parameter. The parameter is a parameter which characterises the thermal load on the primary side, for which a maximum can be determined and which can be deduced from a model of how the system component that is used on the secondary side influences the thermal load on the primary side. In case of a resistive load the heating is determined by the power $P = I^2 *R$. When a constant resistance is assumed, this means that it is proportional to the square of the current $I$. The gradient coils can be approximated as resistive load. The parameter can be the square of the current provided by the primary side during the imaging sequence.

When semiconductor elements are used (e.g. for switching the gradients) such as transistors the thermal losses approximately scale with the average absolute value of the current.

The imaging system is preferably an MR imaging system configured to generate MR images and the time averaged power provided by the primary side during the MR imaging sequence is determined. However, it should be understood that the imaging system may also be a computer tomograph (a CT scanner), a tomosynthesis apparatus or an X-ray apparatus.

The system component can comprise the magnetic field gradients applied in the MR system during the imaging sequence. The utilisation of the magnetic field gradients in the MR imaging sequence may be determined and the time averaged power provided by the primary side to generate the magnetic field gradients in all directions during the imaging sequence can be determined. When the system component considered is the magnetic field gradient, the time period in which the magnetic field gradient is continuously switched is typically in the range of milliseconds. However, the time constant which describes the current or power induced heating of the primary side is typically in the range of several 10 seconds or several minutes. Accordingly, this first time constant is much longer than the time period in which this magnetic field gradient is actually utilised and switched so that it is possible to use the average of the power or of the square of the current provided by the primary side.

The time averaged power may be determined based on the utilisation of the system component and based on a model which translates the temporal variations when using the system component in a load on the primary/supply side needed to drive the system component.

For the magnetic field gradients the model can describe which primary current is needed to generate a magnetic field gradient in a certain direction having a certain gradient strength and a certain time course.

When the sum of the average currents needed in the different gradient directions is known an offset current may also be considered which is flowing in the system independent of the fact whether the magnetic field gradient is actually applied in the imaging sequence or not. Here the model is based on the current that is needed to apply a magnetic field gradient and the offset current. The offset current takes into account the situation that a certain current is flowing to keep the gradient system components or other system components running even when no actual magnetic field gradient is switched.

The model may furthermore take into account at least one parameter alpha which describes the relationship between the applied magnetic field gradient and the current provided by the primary side when the magnetic field gradient is applied. The average of the squared current is then determined taking into account this determined at least one parameter alpha.

Furthermore, the model may use a transfer function which describes how the utilisation of the system component translates into the power provided by the primary side and needed by the secondary side to utilise the system component. The transfer function depends on the system component used and may comprise an exponential transfer function with a time constant in the range of milliseconds. i.e. in the range in which the secondary side uses the power provided by the primary side to switch the system component.

The imaging sequence may be a diffusion imaging sequence used to determine a diffusion property of the object under examination. In diffusion related images higher gradients are used in different gradient directions. Here the heating due to the high currents can reach the temperature limit. With the invention it can be avoided that the temperature limit is actually exceeded.

Furthermore, a plurality of sequential imaging sequences may be used in the MR system, wherein the utilisation of magnetic field gradients applied in the plurality of sequential imaging sequences can be determined taking into account the corresponding time averaged power of each of the plurality of imaging sequences and the maximum of the time averaged current.

During a typical examination, imaging sequences with high magnetic field gradients and low gradients are used. In most cases the time period in which high magnetic field gradients close to their maximum values are used is small compared to the time constant describing the current induced heating. As a consequence, with the above described method the utilisation of magnetic field gradients in the different imaging sequences, especially in the imaging sequences using high magnetic field gradients can be optimized such that the highest possible gradients and thus the shortest image time can be obtained without exceeding the current or temperature limit of the current limiter.

The time averaged power may be determined by averaging the power provided by the primary side over an averaging period T, wherein it is assumed that the averaging period T is larger than the time period in which the system component is switched during the imaging sequence.

Furthermore, the corresponding imaging system is provided which is configured to generate at least one image of the object under examination during the imaging sequence. The system comprises the system component configured to be utilised during the imaging sequence requiring different amounts of power in order to generate the at least one image. The imaging system furthermore comprises the primary side configured to provide the power to the system component resulting in a thermal load on the primary side which must not exceed a predefined temperature limit. Furthermore, the imaging system comprises the secondary side which uses the power provided by the primary side during the imaging sequence. Furthermore, a control unit is provided configured to operate as mentioned above or as discussed in further detail below.

Additionally, a computer program comprising a program code to be executed by the at least one processing unit of an imaging system is provided, wherein the execution of the program code causes the at least one processing unit to execute a method as explained above or as described in further detail below.

Last but not least a carrier comprising the computer program is provided wherein the carrier is one of an electronic signal, optical signal, radio signal or computer readable storage medium.

It should be understood that the features mentioned above and features yet to be explained below can be used not only in the respective combinations indicated, but also in other combinations or in isolation without departing from the scope of the present application. Features of the above mentioned aspects and embodiments described below may be combined with each other in other combinations unless explicitly mentioned otherwise.

Brief description of the drawings

The foregoing and additional features and effects of the application will become apparent from the following detailed description, when read in conjunction with the accompanying drawings in which like reference numerals refer to like elements.

Figure 1 shows a schematic view of an MR system configured to determine an optimized use of a system component such as the magnetic field gradients during an imaging sequence.

Figure 2 shows a schematic view of how a primary side with a current limiter provides the current to a system component provided on a secondary side configured to generate the magnetic field gradients.

Figure 3 shows an example schematic view of a flowchart of a method carried out by the MR system shown in figure 1.

Detailed description

In the following, embodiments of the invention will be described in detail with reference to the accompanying drawings. It should be understood that the following description of embodiments is not to be taken in a limiting sense. The scope of the invention is not intended to be limited by the embodiments described hereinafter or by the drawings, which serve illustrative purposes only.

The drawings are to be regarded as being schematic representations, and elements illustrated in the drawings are not necessarily shown to scale. Rather, the various elements are represented such that their function and general purpose becomes apparent to a person skilled in the art. Any connection or coupling between functional blocks, devices, components of physical or functional units shown in the drawings and described hereinafter may be implemented by an indirect connection or coupling. A coupling between components may be established over a wired or wireless connection. Functional blocks may be implemented in hardware, software, firmware, or a combination thereof.

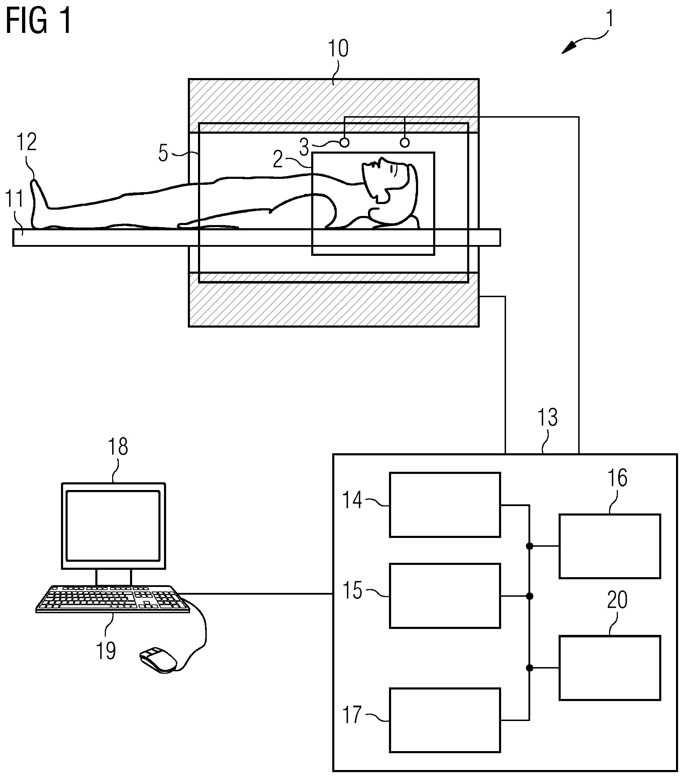

Figure 1 shows a schematic view of an MR system 1 which is configured to provide an operating mode in which the use of a system component such as the RF amplifier or the use of the magnetic field gradients is optimized such that a temperature limit of a current limiter configured to provide the current to the magnetic field gradients is not exceeded.

The MR system 1 comprises a magnet 10 generating a polarization field B0. An object under examination 12 lying on a table 11 is moved into the center of the MR system 1 where MR signals of the RF-excitation can be detected by a receiving coil 2. One example of a gradient coil 5 is shown which is able to generate a magnetic field gradient in one direction. It should be understood that several of the gradient coils may be provided in order to generate the magnetic field gradients in different directions. Furthermore, a transmitting coil 3 is shown configured to transmit RF pulses into the object under examination. By applying RF pulses and magnetic field gradients, the nuclear spins in the object 12 are excited and the currents induced by the relaxation is detected. The way how MR images are generated and how MR signals are detected using a sequence of RF pulses and a sequence of magnetic field gradients are known in the art so that a detailed explanation thereof is omitted.

The MR system 1 comprises a controller 13 which is used for controlling the MR system. The controller or control module 13 comprises a gradient control unit 14 for controlling and switching the magnetic field gradients, an RF control unit 15 for controlling and generating the RF pulses for the imaging sequence. An imaging sequence control unit 16 is provided which controls the sequence of the applied RF pulses and the magnetic field gradients and thus controls the gradient control unit 14 into the RF control unit 15. In a memory 17 computer programs needed for operating the MR system and the imaging sequences necessary for generating the MR images can be stored together with the generated MR images. The generated MR images can be displayed on a display 18 wherein an input unit 19 can be provided used by users of the MR system to control the functioning of the MR system. A processing unit 20 is provided and can coordinate the operation of the different functional units shown in figure 1 and can comprise one or more processors which can carry out instructions stored on the memory 17. The memory 17 can include the program code to be executed by the processing unit 20 so as to implement the invention. As will be described below the controller 13 and/or the processing unit 20 can be configured such that the use of the magnetic field gradients is determined based on a model which employs the application or time course of the magnetic field gradients in the imaging sequence and translates this use into a current provided to the MR system, especially the gradient amplifiers.

The invention will be discussed in connection with the MR imaging system. However it is also possible that the MR system is a spectroscopic MR system which is configured to detect MR signals of the object under examination without actually generating an image, but for generating spectroscopic data of the object under examination. The system component on the primary side could be the RF unit generating the RF pulses needed to excite the MR signal or the gradient unit in case of a single voxel spectroscopy.

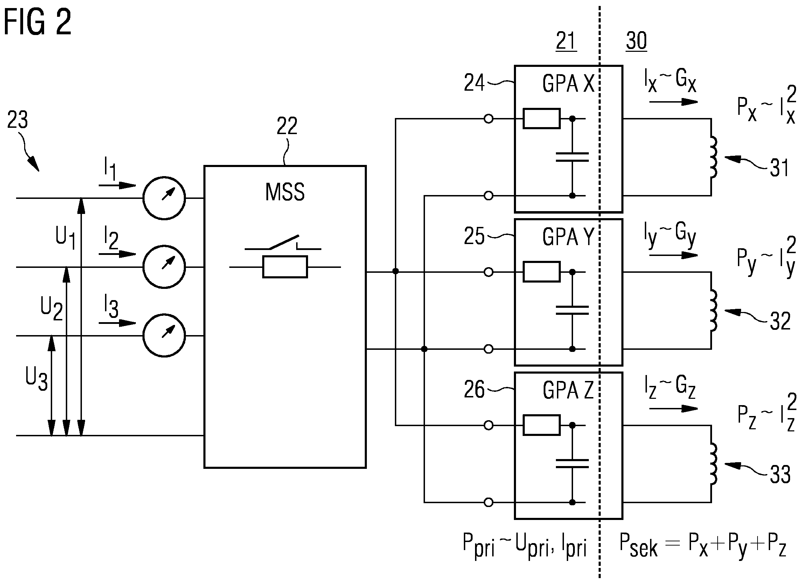

Figure 2 shows a more detailed view of some of the components of the MR system 1 of figure 1. The MR system comprises a primary side 21. The primary side is configured to provide the currents or power needed by the different gradient coils 31, 32 and 33 on the secondary side 30. On the primary side the gradient amplifiers 24, 25 and 26 are shown which provide the currents needed to generate the magnetic field gradients on the secondary side provided by the different gradient coils. The gradient amplifiers 24 to 26 are the elements receiving the power form the primary side and translate it to the secondary side. Figure 2 furthermore shows a protective switch 22 which symbolises that the primary side can only provide current or power up to a certain limit which is mainly due to the heating of components (such as the switch 22 or other components) used at the primary side to generate the currents. Thus switch 22 is not necessarily provided but indicates in the present example that when a certain heat is generated and a certain temperature is reached on the primary side, the primary side stops to provide the current needed to drive the gradient coils 31 to 34 on the secondary side.

The protective switch 22 is configured such that it passes current to the secondary side until a certain temperature limit is reached within the protective switch 22. Thus, the protective switch 22 plays the role of a current limiter which limits the current that can be provided to the secondary side over time. The idea described below is based on the assumption that the time constant that describes the current induced heating of the protective switch 22 or the heating of the primary side itself is much longer than the time period in which the different gradient coils 31, 32 and 33 are actually switched during the imaging sequence to obtain the magnetic field gradients that are needed for the spatial encoding of the signals. There is no need to exactly know the relationship between the actual use of the gradient coils and the thermal heating of the current limiter 22 provided on the primary side. As will be described below a model is used which is capable of determining a load parameter on the primary side that is needed to drive the magnetic field gradient based on the exact switching of the magnetic field gradients as known from the used imaging sequence.

The invention uses the knowledge of a time- averaged power as load parameter that is used by the magnetic field gradient and the maximum time averaged power that can be provided by the primary side over time and based on this knowledge it is possible to determine optimized imaging sequences which can be used by the MR imaging system without actually exceeding the temperature limit on the primary side.

Furthermore, the knowledge may be used how a pause in the imaging sequence influences the heating of the current limiter.

The model described below and discussed above in connection with Figure 2 uses the current source 23, and the protective switch 22 to determine the average current. With the model it is possible to predict the average current that has to be provided by the primary side so that this average current can be compared to the average maximum current which can be provided by the protective switch 22 before a temperature limit of protective switch is reached.

The current needs of this part of the MR system depend on the switching of the magnetic field gradients by the imaging sequence. The switching of the current on the secondary side named as $I_X$, $I_Y$ and $I_Z$ for the three axes X, Y, Z of the gradient system leads to a current on the primary side named $I_1$, $I_2$ and $I_3$ in figure 2 which is distributed by the primary side to the different axes. $I_1$, $I_2$ and $I_3$ describe the 3 phases of a three phase current supply. However other power supplies may be used, and there is not necessarily a relationship between current phase and gradient direction. The use of the gradient amplifiers 24 to 26 can be seen as a power transformation, and might include a temporal filtering of the primary current, by way of example with an exponential transfer function C(t), $C(t) = 1/T_C \exp(-t/T_C)$ in which the time constant TC is typically in the range of 10 - 100 milliseconds. The translation of a magnetic field gradient on the secondary side into a current on the primary side for each of the axes is described by a factor $\alpha(j)$ with j corresponding to the different axes X, Y and Z.

Based on this assumption the square of the current on the primary side $I_{pri}$ which is summed over the three different phases can be described as follows:

$$I^2_{pri}(t) = (I_{Offset} + I_{Grad}(t))^2 \qquad (1)$$

Here, an offset current $I_{offset}$ is assumed which describes the current that is used by the gradient or MR system even when no actual magnetic field gradient is applied in the imaging sequence. Furthermore, a gradient related current $I_{Grad}$ is considered which depends on the actual switching of the magnetic field gradients and which can be mathematically described - assuming a transfer function as explained above - with a convolution as shown in the following equation:

$$I_{Grad}(t) = \Sigma_{j=x,y,z} \, \alpha_j \, G_j^2(t) \otimes C(t) \qquad (2)$$

The above equations show that the load on the primary side is scaling with the fourth power of the current on the secondary side. It is also possible to include alpha into the transfer function and to consider axis-specific transfer functions.

The time constants on the primary side, here the time constants of the protective switch 22 are in the range of several 10 seconds until several minutes and describe the time constant of the current induced heating until a maximum temperature limit is obtained at which the current limiter interrupts the provision of currents. This time constant is much longer than the time period in which the magnetic field gradients are actually switched on which is in the range of milliseconds before they are switched off and switched on again. Accordingly, when considering the relevant thermal load on the primary side, it is sufficient to only take into account average values as shown in the following equation:

$$< I^2_{pri}> = 1/T \int_0^T dt \, I^2_{pri}(t) \qquad (3)$$

The averaging time T used in equation 3 is much longer than the time period in which a single gradient is actually switched on before it is switched off again. The averaging time T is selected such that the resulting average value as determined by equation 3 does not depend on the fact which time period in a temporal evolution of the imaging sequence is actually selected to do the averaging. This time period T is in the same range as the time period describing the current induced heating on the primary side.

The average of the square current as determined by equation 3 is then compared to a maximum value, determined by the maximum temperature under which the protective switch 22 is operating without interrupting the current provided to the secondary side. When the average square of the current is lower than the square of the maximum current, the imaging sequence can get executed with the determined use of the magnetic field gradients. If not, an adaptation of the magnetic field gradients may be necessary, either by introducing further pauses into the imaging sequence or by reducing the amplitude of the magnetic field gradients.

In the example given above actual values of $I_{offset}$ and the parameter $\alpha$ are needed. Those values can be determined directly from the design of the gradient components. If this is not possible or too complex, it is also possible to determine both parameters by calibration measurements. To this end two different currents provided by the primary side are actually measured when different imaging sequences are executed on the secondary side with a known switching pattern of magnetic field gradients. It should be understood that the current used in the two different imaging sequences should differ by a certain amount in order to have different calibration points. With the knowledge of the used magnetic field gradients $G_{x,y,z}(t)$ it is possible to determine the needed parameters. Differences between the different gradient axes may either be determined by averaging based on the assumption that a similar switching pattern is used on each axis or can be determined using a relative scaling when the differences for the different gradient axes are known. Furthermore, it is possible to obtain a separate calibration for each gradient axis.

With this model , the knowledge of the switching of the magnetic field gradients in the imaging sequence and the calibration it is possible to determine the currents that have to be provided by the primary side. If the currents and thus the current induced heating are larger than the threshold provided by the current limiter, the imaging sequence can be adapted accordingly. By way of example it is possible to introduce a pause of a certain time period into the imaging sequence so that the time needed to carry out the imaging sequence is increased. As an alternative, or in addition, the amplitudes of the magnetic field gradients can be reduced.

One possible field of application is the use of the above method in diffusion imaging in which diffusion properties of a certain part of the examined body are determined. For diffusion imaging high magnetic field gradients are required which get successively switch along different directions which can lead to a large load on the primary side. However, as the time periods in which the diffusion gradients are switched on are comparatively short, the above described method can get applied. Accordingly, it is possible to determine in advance whether an image acquisition with the selected gradient switching will be possible without exceeding the temperature limit. This enables the calculation of valid parameter ranges for b-values, echo times TE or repetition times TR, and to limit the selection of parameters to these ranges. The user of the system can thus only select the parameter within the predetermined range which limits the maximum heat load and assures that the temperature limit is not exceeded. In order to stay within the temperature limit it may be necessary to increase the echo time TE which leads to a smaller gradient for a certain b-value and thus to a smaller current, wherein the longer TE may lead to a smaller signal-to-noise ratio. As an alternative it may be necessary to increase the repetition time TR which increases the measurement duration but which also leads to a reduced current below the limit on the primary side.

The aforementioned approach may also be used when several sequential imaging sequences are applied to the object under examination. In an MR imaging of an object under examination, different imaging sequences are used, some of them have high gradient demand whereas others have lower gradient demand. As long as the time periods of the actual switching of the magnetic field gradients is much shorter than the time constant describing the current induced heating and as long as the overall time of the imaging sequences with large magnetic field gradient demand is shorter than the time constant describing the current induced heating, the above described method can increase the performance of the whole MR system.

As far as the calibration is concerned, the calibration can be determined for each individual MR imaging system before delivery or during installation, or for each type of the MR imaging system.

By way of example, the type-specific calibration relating to a certain type of MR imaging systems, e.g. having a certain magnetic field strength or certain gradient components can be carried out in the factory and pre-coded into the system so that a calibration is not necessary each time a system gets installed.

For components which contain complex electric circuitries, it might turn out that the precision of the model predictions depends on the actual type of imaging sequences. By way of example the prediction of the required currents with high precision might be possible either for sequences with strong magnetic field gradient variations or for nearly constant magnetic field gradients, and this may depend on the fact whether the calibration was obtained with one or the other type of sequences. In these cases it may be possible to determine the calibration, especially for this class of imaging sequences for which the reaching of the temperature limit is highly likely. Furthermore, it is possible to carry out different calibrations for the different classes of imaging sequences.

If the MR systems should be used in such a way that the user can only select imaging parameters within a parameter range by which the limits of the current limiter is not exceeded, it may be necessary to determine and predict the temperature induced heating very quickly. To this end it may be possible to make certain assumptions for the calculation of the magnetic field gradients. By way of example, a normal gradient pulse may have a trapezoidal form with an amplitude G, a ramp time $T_R$ and a constant maximum value during $T_D$. These trapezoidal shapes can be simplified with a gradient having a square shape with a certain amplitude G and a switching time $T_K$. The time period $T_K$ may be determined based on $T_R$ and $T_D$ such that the resulting current need corresponds to the actual use of the currents. By way of example time period $T_K$ may be determined as follows:

$$T_K = T_R * 2/3 + T_D \qquad\qquad (4)$$

Furthermore, it is possible to take into account the use of a pause within the imaging sequence. If the average load is described as an integral over the load B(t), then $\underline{B}$ is determined as follows:

$$\underline{B} = 1/T \;{}_0\!\int^T dt\; B(t) \qquad\qquad (5)$$

A pause of the duration TP leads to the following average load $\underline{B}$':

$$\underline{B}' = 1/(T + TP) \;{}_0\!\int^{T+TP} dt\; B(t) = 1/(T + TP) \;{}_0\!\int^T dt\; B(t) = \underline{B}\; T\;/\;(T + TP) \qquad\qquad (6)$$

Based on the knowledge of the average load $\underline{B}$ and the time period T, it is possible to directly determine the pause needed to stay below the limit $B_{max} \geq \underline{B}'$.

The way how a pause influences the mean load can be determined numerically. It is possible to introduce longer and shorter pauses into the imaging sequence and then to determine the average load.

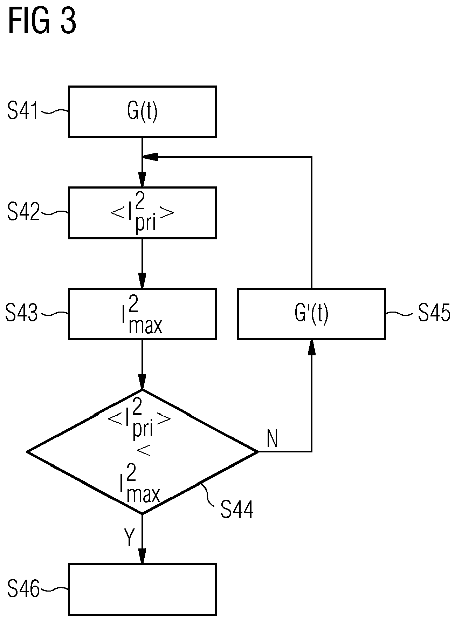

Figure 3 summarizes some of the steps carried out to determine a use of the system components such as the magnetic field gradients in an imaging system. In step S41 the use of the system component during the imaging sequence is determined. In the example given above it is the exact switching of the different magnetic field gradients over time during the imaging sequence. In step S42 the square of the average current provided by the primary side for the determined use is calculated, e.g. based on a model which can translate the used gradient into a current provided by the primary side. In the example above the model is described in the above equations (1) to (3). Furthermore, in step S43 the square of the maximum current that can be provided by the primary side without overheating the primary side is determined. The maximum value can be either determined from data sheets of the manufacturer of the installed electric components or may be determined based on experiments in which for a defined gradient switching the amplitude of the gradient is increased step by step while detecting the current or power provided by the primary side. When the system switches off or when a measured temperature of a component exceeds the limit specified by its manufacturer, the maximum average current or power is found. In step S44 it is determined whether the average current is smaller than the maximum current wherein the determination is based on the square of the two values. If this is not the case the use of the system component has to be adapted in step S45 as symbolized by G'(T) and the calculation of steps S42 to S44 is repeated with the adapted use of the system component. If step S44 indicates that the temperature limit is not reached the imaging sequence can be used in step S46 with the determined use of the gradient as determined in step S41. In a further embodiment it may also be determined in step S44 how close the square of the primary current is to the square of the maximum current and the imaging sequence may be adapted such that either the time is minimized that is used for applying the imaging sequence with the conditions that the square of the primary current is smaller than the square of the maximum current limit. As an alternative, the imaging sequence may be such that a certain quality parameter such the signal-to-noise ratio, contrast-to-noise ratio in a certain part of the image is optimized by using larger magnetic field gradients or higher values of the system component without exceeding the current limit.

Summarizing the above described method makes it possible to operate the MR system close to the temperature limits without exceeding them. Accordingly, the imaging system can be operated without interrupting the measurements due to the fact that temperature limits by the system components have been exceeded.

The use of the average values makes it possible to model very complex relationships. Furthermore, as discussed, the current provided by the primary side is determined based on a model. This model may additionally use the characteristics of the gradient amplifier by using calibration measurements in order to determine a relation between the current used by the secondary side to switch the system component and the current provided by the primary side.

**Claims**

1. A method for determining a utilisation of a system component (31-33) in an imaging system (1) in which at least one image of an object under examination is generated during the imaging sequence, the imaging system comprising a primary side (21) configured to provide power to the system component resulting in a thermal load on the primary side which must not exceed a predefined temperature limit, and a secondary side (30) comprising the system component (31-33) which utilises the power provided by the primary side during the image sequence, the method comprising:

- determining the utilisation of the system component (31-33) during the imaging sequence,
- determining a time averaged power supplied by the primary side during the imaging sequence with the determined utilisation,
- determining a maximum time averaged power that can be supplied by the primary side (21) over a duration of at least one imaging sequence while not exceeding the predefined temperature limit,
- determining whether the time averaged power is smaller than the maximum time averaged power, wherein when the time averaged power is not smaller than the maximum time averaged power, adapting the use of the system component during the imaging sequence until the time averaged power is smaller than the maximum time averaged power.

2. The method according to claim 1 wherein the time averaged power is determined based on an assumption that a first time constant describing a power induced heating of the primary side when power is supplied to the system component (31-33) is much longer than a time period in which the system component (31-33) is continuously utilised during the imaging sequence.

3. The method according to claim 1 or 2, wherein the utilisation of the system component (31-33) is determined without the knowledge of a relationship of how the use of the system component during the imaging sequence influences a heating of the primary side.

4. The method according to any of the preceding claims, further determining an optimized imaging sequence based on the comparison of the time averaged power to the maximum time averaged power such that at least one of the following parameters is optimized for the imaging sequence: a time needed to generate the at least one image and an image quality parameter of the at least one image.

5. The method according to any of the preceding claims, wherein determining the time averaged power comprises determining an average of a parameter describing the heating of the primary side when power is provided to the system component, and determining the maximum time averaged power comprises determining a maximum of the average of the parameter.

6. The method according to claim 5, wherein the parameter is a square of the current provided by the primary side during the imaging sequence.

7. The method according to any of the preceding claims, wherein the imaging system (1) is an MR system configured to generate MR images, wherein the time averaged power provided by the primary side (21) during the MR imaging sequence is determined when the time averaged power is determined.

8. The method according to claim 7, wherein the system component (31-33) comprises a gradient field generating unit used to generate magnetic field gradients applied in the MR system, wherein the utilisation of the magnetic field gradients in the MR imaging sequence is determined and the average of square current provided by the primary side to set up the magnetic field gradients during the imaging sequence is determined.

9. The method according to claim 7 or 8, wherein the square current is determined taking into account an offset current which is flowing independent of the fact whether the magnetic field gradients are applied in the imaging sequence.

10. The method according to claim 8 or 9, further comprising the step of determining at least one parameter $\alpha$ describing a relationship between the applied magnetic field gradients and the current provided by the primary side when the magnetic field gradient is applied, wherein the average of the square current is determined taking into account the determined at least one parameter $\alpha$.

11. The method according to any of claims 7 to 10, wherein the imaging sequence is a diffusion imaging sequence used to determine a diffusion property in the object under examination.

12. The method according to any of claims 7 to 11, wherein a plurality of sequential imaging sequences are used in the MR system, wherein the utilisation of the magnetic field gradients applied in the plurality of sequential imaging sequences is determined taking into account the corresponding time averaged power for each of the plurality of imaging sequences and the maximum time averaged power.

13. The method according to any of the preceding claims, wherein the time averaged power is determined by averaging the power provided by the primary side (21) over an averaging period T, wherein it is assumed that the averaging period is larger than the time period in which the system component is continuously switched during the imaging sequence.

14. The method according to any of the preceding claims wherein the time averaged power is determined based on the utilisation of the system component based on a model which translates the utilisation of the system component in the power needed to use the system component.

15. An imaging system (1) configured to generate at least one image of an object under examination during an imaging sequence, the system comprising:

   - a system component (31-33) configured to be switched on and off during the imaging sequence in order to generate the at least one image,
   - a primary side (21) configured to provide power to the system component, resulting in a thermal load on the primary side which must not exceed a predefined temperature limit,
   - a secondary side (30) comprising the system component (31-33) which uses the power provided by the primary side during the imaging sequence,
   - a control unit (13, 20) configured to:

      - determine the utilisation of the system component (31-33) during the imaging sequence,
      - determine a time averaged power supplied by the primary side during the imaging sequence with the determined utilisation,
      - determine a maximum time averaged power that can be supplied by the primary side (21) over a duration of at least one imaging sequence while not exceeding the predefined temperature limit,
      - determine whether the time averaged power is smaller than the maximum time averaged power, wherein when the time averaged power is not smaller than the maximum time averaged power, adapting the use of the system component during the imaging sequence until the time averaged power is smaller than the maximum time averaged power.

16. The imaging system according to claim 15, further being configured to operate according to a method as mentioned in any of claims 2 to 14.

17. A computer program comprising program code to be executed by at least one processing unit of an imaging system, wherein execution of the program code causes the at least one processing unit to execute a method according to any of claims 1 to 14.

18. A carrier comprising the computer program of claim 17, wherein the carrier is one of an electronic signal, optical signal, radio signal, or computer readable storage medium.

FIG 1

FIG 2

# FIG 3

Europäisches
Patentamt

European
Patent Office

Office européen
des brevets

**EUROPEAN SEARCH REPORT**

Application Number

EP 18 20 6297

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP 2004 357771 A (GE MED SYS GLOBAL TECH CO LLC) 24 December 2004 (2004-12-24) <br> * paragraphs [0005], [0006], [0012], [0013], [0017], [0022], [0025], [0026], [0032] - [0062]; figures 1-4 * | 1-18 | INV. <br> G01R33/54 <br> G01R33/385 |
| X | WO 2018/002126 A1 (KONINKLIJKE PHILIPS NV [NL]) 4 January 2018 (2018-01-04) <br> * page 1, line 24 - page 2, line 12 * <br> * page 3, line 24 - page 4, line 19 * <br> * page 5, line 23 - page 7, line 21 * <br> * page 11, line 24 - page 12, line 15 * <br> * page 15, line 24 - page 16, line 33 * <br> * page 19, line 27 - page 21, line 20; figures 1, 4 * | 1-18 | |
| A,D | DE 10 2013 204310 A1 (SIEMENS AG [DE]) 18 September 2014 (2014-09-18) <br> * abstract * <br> * paragraphs [0016], [0023], [0028], [0041], [0046], [0049], [0050]; claim 1 * | 1-18 | |

TECHNICAL FIELDS SEARCHED (IPC)

G01R

The present search report has been drawn up for all claims

| Place of search | Date of completion of the search | Examiner |
|---|---|---|
| Munich | 17 June 2019 | Faber-Jurk, Sonja |

EPO FORM 1503 03.82 (P04C01)

## ANNEX TO THE EUROPEAN SEARCH REPORT
## ON EUROPEAN PATENT APPLICATION NO.

EP 18 20 6297

This annex lists the patent family members relating to the patent documents cited in the above-mentioned European search report.
The members are as contained in the European Patent Office EDP file on
The European Patent Office is in no way liable for these particulars which are merely given for the purpose of information.

17-06-2019

| Patent document cited in search report | Publication date | Patent family member(s) | Publication date |
|---|---|---|---|
| JP 2004357771 A | 24-12-2004 | JP 4305736 B2<br>JP 2004357771 A | 29-07-2009<br>24-12-2004 |
| WO 2018002126 A1 | 04-01-2018 | CN 109477874 A<br>EP 3475717 A1<br>WO 2018002126 A1 | 15-03-2019<br>01-05-2019<br>04-01-2018 |
| DE 102013204310 A1 | 18-09-2014 | CN 104049545 A<br>DE 102013204310 A1<br>JP 6092142 B2<br>JP 2014176685 A<br>KR 20140112452 A<br>US 2014278195 A1 | 17-09-2014<br>18-09-2014<br>08-03-2017<br>25-09-2014<br>23-09-2014<br>18-09-2014 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DE 102008015261 B4

- DE 102013204310 A1